(19) Europäisches Patentamt
European Patent Office
Office européen des brevets

(11) Publication number : **0 442 757 A2**

# (12) EUROPEAN PATENT APPLICATION

(21) Application number : **91301243.1**

(22) Date of filing : **15.02.91**

(51) Int. Cl.$^5$ : **A61K 31/70**, A61K 31/71, C07H 19/06

(30) Priority : **16.02.90 GB 9003543**

(43) Date of publication of application : **21.08.91 Bulletin 91/34**

(84) Designated Contracting States : **AT BE CH DE DK ES FR GB GR IT LI LU NL SE**

(71) Applicant : **THE WELLCOME FOUNDATION LIMITED**
**Unicorn House 160 Euston Road**
**London NW1 2BP (GB)**

(72) Inventor : **Rahim, Saad George**
**The Wellcome Research Laboratories, Langley Court**
**Beckenham, Kent, BR3 3BS (GB)**
Inventor : **Bogunovic-Batchelor, Mirjana Vladimira**
**The Wellcome Research Laboratories, Langley Court**
**Beckenham, Kent, BR3 3BS (GB)**
Inventor : **Tranter, George Edward**
**The Wellcome Research Laboratories, Langley Court**
**Beckenham, Kent, BR3 3BS (GB)**

(74) Representative : **Garrett, Michael et al**
**The Wellcome Research Laboratories Group Patents and Agreements Langley Court**
**Beckenham Kent BR3 3BS (GB)**

(54) **Therapeutic nucleosides.**

(57) The present invention relates to 3'-fluoro nucleoside analogues and their use in medical therapy, particularly for the treatment or prophylaxis of human immunodeficiency virus and hepatitis B virus infections, to methods for their preparation and to formulations containing them.

EP 0 442 757 A2

# THERAPEUTIC NUCLEOSIDES

The present invention relates to certain 3′-fluoro nucleoside analogues, pharmaceutically acceptable derivatives thereof, and the use of such analogues and derivatives compounds in therapy, particularly for the treatment or prophylaxis of certain viral infections.

One group of viruses which has recently assumed a particular importance are the retroviruses. Retroviruses form a sub-group of RNA viruses which, in order to replicate, must first 'reverse transcribe' the RNA of their genome into DNA ('transcription' conventionally describes the synthesis of RNA from DNA). Once in the form of DNA, the viral genome may be incorporated into the host cell genome, allowing it to take advantage of the host cell's transcription/translation machinery for the purposes of replication. Once incorporated, the viral DNA is virtually indistinguishable from the host's DNA and, in this state, the virus may persist for the life of the cell.

A species of retrovirus, Human Immunodeficiency Virus (HIV), has been reproducibly isolated from humans with Acquired Immune Deficiency Syndrome (AIDS) or with the symptoms that frequently precede AIDS. AIDS is an immunosuppressive or immunodestructive disease that predisposes subjects to fatal opportunistic infections. Characteristically, AIDS is associated with a progressive depletion of T-cells, especially the helper-inducer subset bearing the $OKT^4$ surface marker. HIV is cytopathic and appears to preferentially infect and destroy T-cells bearing the $OKT^4$ marker and it is now generally recognised that HIV is the etiological agent of AIDS.

Since the discovery that HIV is the etiological agent of AIDS, numerous proposals have been made for anti-HIV chemotherapeutic agents that may be effective in treating AIDS. Thus, for example, European Patent Specification No. 196185 describes 3′-azido-3′-deoxythymidine (which has the approved name zidovudine), its pharmaceutically acceptable derivatives and their use in the treatment of human retrovirus infections including AIDS and associated clinical conditions. Other nucleoside derivatives that have been suggested for the treatment of HIV infections include the 3′-fluoronucleosides described for example in European Patent No. 0254 268 and International Patent Specification 88/0050.

Another group of viral pathogens of major consequence worldwide are the hepatitis viruses, in particular hepatitis B virus (HBV). HBV is most common in Asian countries, and prevalent in sub-Saharan Africa. The virus is etiologically associated with primary hepatocellular carcinoma and is thought to cause 80% of the world's liver cancer. In the United States more than ten thousand people are hospitalised for HBV illness each year, and an average of 250 die with fulminant disease. The United States currently contains an estimated pool of 500,000-1 million infectious carriers. Chronic active hepatitis will develop in over 25% of carriers, and often progresses to cirrhosis. It is estimated that 5000 people die from HBV related cirrhosis each year in the USA, and that perhaps 1000 die from HBV-related liver cancer. Thus, there is a great need for effective antiviral agents, both to control the chronic infection and reduce progression to hepatocellular carcinoma.

Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease as outlined above. In "Viral Infections of Humans" (second edition, Ed., Evans, A.S. (1982) Plenum Publishing Corporation, New York), Chapter 12 describes the etiology of viral hepatitis infections.

We have now surprisingly discovered that 3′-fluoro-2′,3′-dideoxynucleosides as referred to below have potent activity against retroviruses such as HIV, as well as HBV.

According to the present invention there is provided the use of 3′-fluoro-2′,3′-dideoxynucleosides of formula (I).

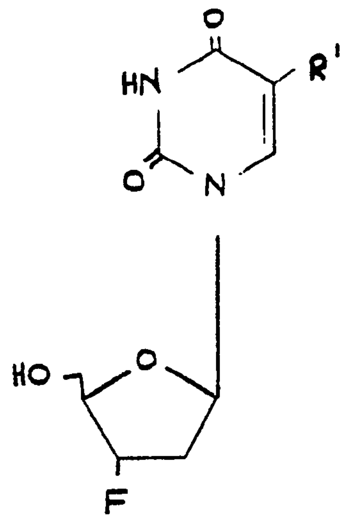

(I)

wherein $R^1$ represents a $C_{1-4}$alkylthiomethyl (e.g. methylthiomethyl, or ethylthiomethyl), $C_{1-4}$ alkoxymethyl (e.g. methoxymethyl, ethoxymethyl), cyanomethyl, azidomethyl, mono-, di- or tri halomethyl (e.g. trifluoromethyl), nitro, 2-haloalkynyl (e.g. 2-chloroethynyl), aminomethyl, $C_{1-4}$ alkoxy (e.g. methoxy) or $C_{1-4}$ alkylthio (e.g. methylthio) group ; or a pharmaceutically acceptable derivative thereof in the manufacture of a medicament for the treatment or prophylaxis of a retroviral infection, particularly a human immunodeficency virus infection; or a hepatitis B viral infection.

The compounds of formula (I) and their pharmaceutically acceptable derivatives are hereinafter referred to as compounds according to the invention.

The term "alkyl" is used herein to describe straight chain or branched chain alkyl groups.

In a further aspect of the present invention there is included a method for the treatment of a viral infection, particularly retroviral or hepatitis B viral infections of a mammal including humans which comprises treating the mammal with an antivirally effective amount of a compound according to the invention.

Compounds of formula (I) wherein $R^1$ is azidomethyl or aminomethyl are disclosed in East German patent application DD268475, which refers to these compounds as virustatic agents

Compounds of formula (I) wherein $R^1$ is methylthiomethyl, methoxymethyl or trifluoromethyl are generically embraced by the disclosure in European Patent Applications No. 0322384, but are not specifically disclosed therein. We have now found that such compounds exhibit advantageous properties, including particularly potent anti-HIV activity. On this basis, the use of these compounds in the treatment of HIV-related conditions constitutes a further aspect of the present invention.

European patent No. 0254 268 discloses a compound of formula (I) wherein $R^1$ represents bromomethyl as an intermediate for the preparation of certain 3′-fluoronucleosides, but without any reference to the medical utility of this compound.

Compounds of formula (I) wherein $R^1$ represents $C_{2-4}$ alkylthiomethyl or $C_{2-4}$ alkoxymethyl are also included in the present invention.

The present invention further includes 3′-fluoro-2′,3′-dideoxynucleosides of formula (IA)

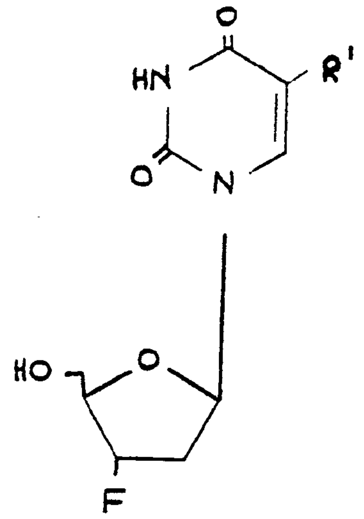

(IA)

wherein $R^1$ represents a $C_{1-4}$ alkylthiomethyl (e.g. methylthiomethyl or ethylthiomethyl), $C_{1-4}$ alkoxymethyl (e.g. methoxymethyl, ethoxymethyl), cyanomethyl, mono-, di- or tri-haloalkyl (e.g. trifluoromethyl), nitro, 2-haloalkynyl (e.g. 2-chloroethynyl), $C_{1-4}$ alkoxy (e.g. methoxy) or $C_{1-4}$ alkylthio (e.g. methylthio) group ; or a pharmaceutically acceptable derivative thereof for use in medical therapy, in particular for the treatment or prophylaxis of viral infections especially retroviral and hepatitis B viral infections.

Examples of retroviral infections which may be treated in accordance with the invention include human retroviral infections such as HIV-I, HIV-2 and Human T-cell Lymphotropic Virus (HLTV) e.g. HTLV-I or HTLV-II infections.

The compounds according to the invention are also useful for the treatment of clinical conditions associated with retroviral infections, for example, AIDS, Kaposi's sarcoma, thrombocytopenic purpura, AIDS-related complex (ARC), progressive generalized lymphadenopathy (PGL), and patients carrying HIV-antibodies or who are seropositive to the HIV virus, as well as chronic neurological conditions such as multiple sclerosis or tropical spastic paraparesis.

The compounds according to the invention may also be used for the treatment or prophylaxis of infections carried by DNA viruses which, like retroviruses, are incorporated into the host genome during their life-cycle, i.e. DNA viruses such as hepatitis B. Thus, there is further provided the compounds according to the invention for use in the treatment or prophylaxis of infections caused by such retrovirus-like viruses.

The present invention further provides as novel compounds the compounds of formula (IB)

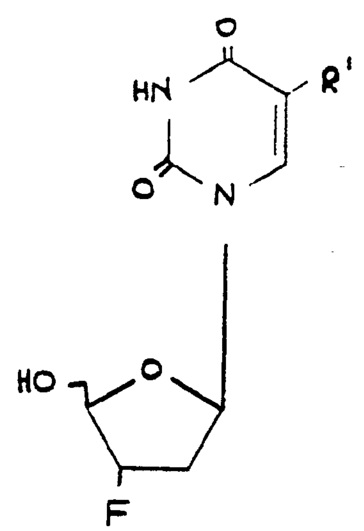

(IB)

wherein $R^1$ represents a $C_{1-4}$ alkylthiomethyl (e.g. methylthiomethyl or ethylthiomethyl), $C_{1-4}$ alkoxymethyl (e.g. methoxymethyl, ethoxymethyl), cyanomethyl, mono- di- or tri-halomethyl (other than bromomethyl, e.g. trifluoromethyl), nitro, 2-hakoalkynyl (e.g. 2-chloroethynyl), $C_{1-4}$ alkoxy (e.g. methoxy) and $C_{1-4}$ alkylthio (e.g.

methylthio) group ; or a pharmaceutical acceptable derivative thereof.

Examples of compounds of formula (IB) above include the following compounds of formula (IC)

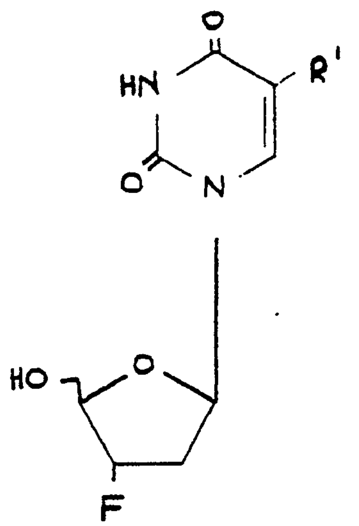

(IC)

wherein $R^1$ represents a cyanomethyl, di-halomethyl, nitro, 2-haloalkynyl (e.g. 2-chloroethynyl), $C_{1-4}$ alkoxy (e.g. methoxy) and $C_{1-4}$ alkylthio (e.g. methylthio) group ; or a pharmaceutical acceptable derivative thereof.

Further examples of compounds of formula (IB) above include the compounds of formula (ID)

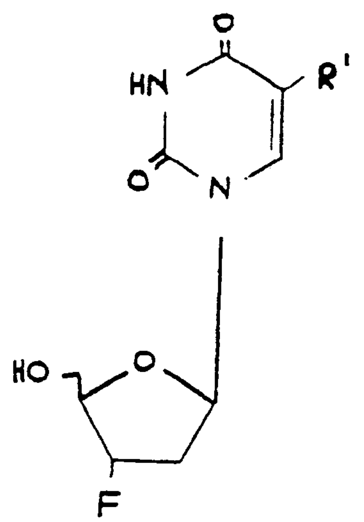

(ID)

wherein $R^1$ represents a cyanomethyl, nitro, 2-haloalkynyl (e.g. 2-chloroethynyl), $C_{1-4}$ alkoxy (e.g. methoxy) and $C_{1-4}$ alkylthio (e.g. methylthio) group ; or a pharmaceutical acceptable derivative thereof.

Formulae (I), (IA), (IB), (IC) and (ID) above depict the compound in the keto tautomeric form. It will be appreciated that the compound may also exist in the corresponding enol tautomeric form.

Preferred compounds of formula (IB) include those wherein $R^1$ represents a $C_{1-4}$ alkylthiomethyl or $C_{1-4}$ alkoxymethyl ; or a pharmaceutically acceptable derivative thereof.

Particularly preferred compounds of formula (IB) include :-

(1) 2′,3′-dideoxy-3′-fluoro-5-methoxymethyluridine

(2) 2′,3′-dideoxy-3′-fluoro-5-ethoxymethyluridine

(3) 2′,3′-dideoxy-3′-fluoro-5-methylthiomethyluridine

By "a pharmaceutically acceptable derivative" is meant any pharmaceutically acceptable salt, ester, or salt of such ester, of a compound of formula (I) or any other compound which, upon administration to the recipient, is capable of providing (directly or indirectly) such a compound or an antivirally active metabolite or residue thereof.

Preferred esters of the compounds of formula (I) include carboxylic acid esters in which the non-carbonyl

moiety of the ester grouping is selected from straight or branched chain alkyl (e.g. methyl, n-propyl, n-butyl or t-butyl), alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy or amino) ; sulphonate esters such as alkyl- or alkylarylsulphonyl (e.g. methanesulphonyl) ; amino acid esters (e.g. L-valyl or L-isoleucyl) ; and mono-, di- or tri-phosphate esters. In such esters unless otherwise specified, any alkyl moiety present advantageously contains 1 to 18 carbon atoms, particularly 1 to 4 carbon atoms. Any aryl moiety present in such esters advantageously comprises a phenyl group. Any reference to any of the above compounds also includes a reference to a pharmaceutically acceptable salt thereof.

Examples of pharmaceutically acceptable salts of the compound of formula (I) and pharmaceutically acceptable derivatives thereof include base salts, e.g. derived from an appropriate base, such as alkali metal (e.g. sodium), alkaline earth metal (e.g. magnesium) salts, ammonium and $NX^{+}_{4}$ (wherein X is $C_{1-4}$ alkyl). Physiologically acceptable salts of an hydrogen atom or an amino group include salts of organic carboxylic acids such as acetic, lactic, tartaric, malic, isethionic, lactobionic and succinic acids ; organic sulfonic acids such as methanesulfonic, ethanesulfonic, benzenesulfonic and p-toluenesulfonic acids and inorganic acids such as hydrochloric, sulfuric, phosphoric and sulfamic acids. Physiologically acceptable salts of a compound of an hydroxy group include the anion of said compound in combination with a suitable cation such as $Na^+$, $NH_4^+$, and $NX_4^+$ (wherein X is a $C_{1-4}$ alkyl group).

The compounds according to the invention may be employed in combination with other therapeutic agents for the treatment of the above infections or conditions. Examples of such further therapeutic agents include agents that are effective for the treatment of HIV infections or associated conditions such as 3′-azido-3′-deoxythymidine (zidovudine), other 2′,3′-dideoxynucleosides such as 2′,3′-dideoxycytidine, 2′,3′-dideoxyadenosine and 2′,3′-dideoxyinosine, carbovir, acyclic nucleosides (e.g. acyclovir), 2′,3′-didehydrothymidine, interferons such as α-interferon, renal excretion inhibitors such as probenicid, nucleoside transport inhibitors such as dipyridamole, as well as immunomodulators such as interleukin II and granulocyte macrophage colony stimulating factors, erythropoetin, phosphonoformic acid and soluble $CD_4$ and genetically engineered derivatives thereof. The component compounds of such combination therapy may be administered simultaneously, in either separate or combined formulations, or at different times, e.g. sequentially, such that a combined effect is achieved.

The compounds according to the invention, also referred to herein as active ingredients, may be administered for therapy by any suitable route including oral, rectal, nasal, topical (including buccal and sublingual), vaginal, parenteral (including subcutaneous, intramuscular, intravenous and intradermal) and pulmonary. It will also be appreciated that the preferred route will vary with the condition and age of the recipient, the nature of the infection and the chosen active ingredient.

In general a suitable dose for each of the above named antiviral infections e.g. HIV, HBV is in the range of 0.5 to 120 mg per kilogram body weight of the recipient per day, preferably in the range of 1 to 90 mg per kilogram body weight per day and most preferably in the range 2 to 60 mg per kilogram body weight per day. The desired dose is preferably presented as two, three, four, five, six or more sub-doses administered at appropriate intervals throughout the day. These sub-doses may be administered in unit dosage forms, for example, containing 1 to 1500 mg, preferably 5 to 1000 mg, and most preferably 10 to 700 mg of active ingredient per unit dosage form.

Ideally, the active ingredient should be administered to achieve peak plasma concentrations of the active compound of from about 0.25 to about 100 μM, preferably about 0.5 to 70 μM, most preferably about 1 to about 50 μM. This may be achieved, for example, by the intravenous injection of a 0.1 to 5% solution of the active ingredient, optionally in saline, or orally administered, for example, as a tablet, capsule or syrup containing about 0.5 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible for the active ingredient to be administered alone it is preferable to present it as a pharmaceutical formulation comprising at least one active ingredient, as defined above, together with one or more pharmaceutically acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) adminstration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient ; as a powder or granules ; as a solution or suspension in an aqueous or non-aqueous liquid ; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, crossed-linked sodium carboxmethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example hydroxypropylmethyl cellulose in varying proportions to provide the desired release profile. Tablets may optionally be provided with an enteric coating, to provide release in parts of the gut other than the stomach.

Formulations suitable for topical administration in the mouth include lozenges comprising the active ingredient in a flavoured basis, usually sucrose and acacia or tragacanth ; pastilles comprising the active ingredient in an inert basis such as gelatin and glycerin, or sucrose and acacia ; and mouthwashes comprising the active ingredient in a suitable liquid carrier.

Pharmaceutical compositions for topical administration according to the present invention may be formulated as an ointment, cream, suspension, lotion, powder, solution, paste, gel, spray, aerosol or oil. Alternatively, a formulation may comprise a dressing such as a bandage or adhesive plaster impregnated with active ingredients and optionally one or more excipients or diluents. Carriers which may be used include e.g. polyhydric alcohols such as polyethylene glycols, propylene glycol or glycerol. Suitable excipients are those known in the art to be appropriate.

Formulations for rectal administration may be presented as a suppository with a suitable base comprising for example cocoa butter or a salicylate.

Formulations suitable for vaginal administration may be presented as pessaries, tampons, creams, gels pastes, foams or spray formulations containing in addition to the active ingredient such carriers as are known in the art to be appropriate.

Formulations suitable for parenteral administration include aqueous and non-aqueous isotonic sterile injections solutions which may contain anti-oxidants, buffers, bacteriostats and solutes which render the formulation isotonic with the blood of the intended recipient ; and aqueous and non-aqueous sterile suspensions which may include suspending agents and thickening agents, and liposomes or other microparticulate systems which are designed to target the compound to blood components or one or more organs. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example, those suitable for oral administration may include such further agents as sweeteners, thickeners and flavouring agents.

The compounds according to the invention may also be presented for the use in the form of veterinary formulations, which may be prepared, for example, by methods that are conventional in the art.

The compounds of formula (I) and their pharmaceutically acceptable derivatives may be prepared for example by a process which comprises :-

(A) for the preparation of compounds of formula (I) wherein $R^1$ represents a $C_{1-4}$alkylthiomethyl, $C_{1-4}$alkoxymethyl, cyanomethyl, azidomethyl, mono-, di- or tri-halomethyl, nitro, 2-haloalkynyl or aminomethyl group, removing a protecting group from a compound of formula (II)

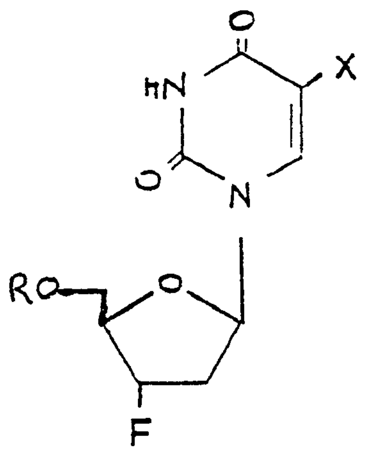

(II)

(wherein OR is a protected hydroxy group and X has the same meaning as $R^1$ in the resulting compounds of formula (I) ;
(B) for the preparation of compounds of formula (I) wherein $R^1$ represents a $C_{1-4}$ alkoxy group, reacting a compound of formula (IV)

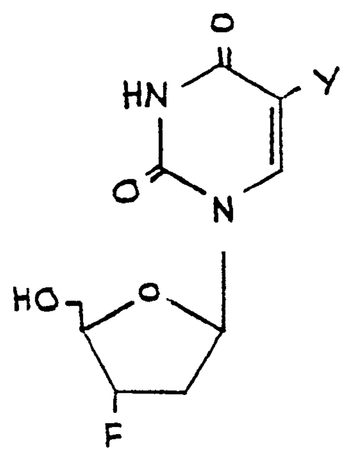

(IV)

(wherein Y is a hydroxy group) with an agent(s) and/or under such conditions as serve to alkylate the 5-hydroxy group on the uracil base.
(C) for the preparation of compounds of formula (I) wherein $R^1$ represents a $C_{1-4}$ alkylthio group, reacting a compound of formula (IV) wherein Y represents a leaving group with an agent(s) and/or under such conditions serving to introduce a $C_{1-4}$ alkylthio group at the 5-position.
(D) reacting a compound of formula (V) :

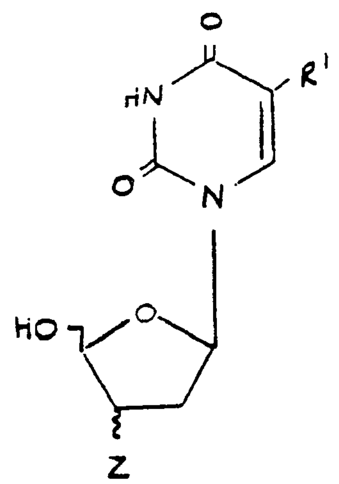

(V)

(wherein $R^1$ is as hereinbefore defined and Z represents a precursor group for the fluoro group) with an agent(s) and/or under such conditions serving to convert the said precursor group to a fluoro group ; or (E) reacting a pyrimidine base of formula (VI) :

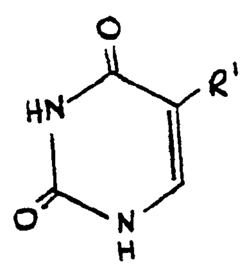

(VI)

(wherein $R^1$ is as hereinbefore defined) or a functional equivalent thereof, with a compound serving to introduce the desired ribofuranosyl ring at the 1-position of the pyrimidine base of formula (VI) ;

and thereafter, or simultaneously therewith, effecting one or more of the following optional conversions :-

(i) removing any remaining protecting groups ;

(ii) when a compound of formula (I) is formed, converting it into a pharmaceutically acceptable derivative thereof ;

(iii) when a pharmaceutically acceptable derivative of a compound of formula (I) is formed, converting the said derivative into a compound of formula (I), or a different derivative thereof.

The present invention includes a process for the preparation of compounds of formula (IB) and their pharmaceutically acceptable derivatives in accordance with the process described above for the preparation of compounds of formula (I) and their pharmaceutically acceptable derivatives.

In the above-described process according to the invention it will be appreciated that the starting compounds of formulae (II), (III), (IV), (V) and (VI), as well as the above-mentioned agents and conditions, will be selected from those that are known in the art of nucleoside synthetic chemistry. For example as described in Nucleic Acid Chemistry : Improved New Synthetic Procedures, Methods and Techniques. Ed. L.B. Townsend and R.S. Tipson-Wiley Interscience (1978) and Nucleoside Analogues : Chemistry, Biology and Medical Applications, Ed. R.T. Walker, E. de Clercq and F. Eckstein, NATO Advanced Study Instituted, Plenum press (1979). Examples of such conversion procedures are described hereinafter for guidance and it will be understood that they can be modified in conventional manner depending on the desired compound of formula (I). In particular, where a conversion is described which would otherwise result in the undesired reaction of labile groups then such groups may be protected in conventional manner, with subsequent removal of the protecting groups after completion of the conversion.

In process A the protected hydroxy group (OR) may be for example an ester grouping particularly $C_{1-6}$ alkanoyloxy (e.g. acetyloxy) or aroyloxy or alkaroyloxy (e.g. toluoyloxy), or an alkoxycarbonyloxy (e.g. methoxycarbonyloxy) ; or an ether group such as trialkylsilyloxy (e.g. t-butyldimethylsilyloxy) or an aralkyloxy group (e.g. triphenylmethyloxy) ; or a phosphate group.

Such groups may be converted by for example hydrolysis to the desired hydroxy compound. A particularly

preferred hydroxy protecting group is the acetyloxy group which may, for example, be hydrolysed under basic conditions, for example, with sodium methoxide/methanol, aqueous methylamine or ammonia. The group may alternatively be hydrolysed enzymatically, for example, by esterase.

Another preferred hydroxy protecting group is t-butyldimethylsilyloxy group which may be removed by for example acid hydrolysis or using tetrabutylammoniumfluoride (TBAF).

In process A the starting compound of formula (II) wherein X is $C_{1-4}$ alkylthiomethyl, $C_{1-4}$ alkoxymethyl, cyanomethyl, azidomethyl, or aminomethyl may conveniently be prepared from compounds of formula (III)

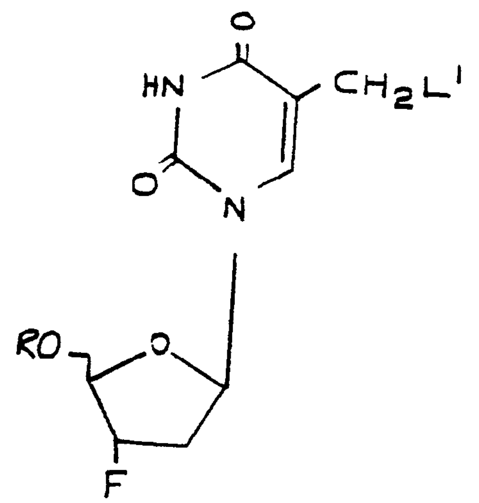

(III)

(wherein OR is as hereinbefore defined and $L^1$ is a suitable leaving group other than hydroxy, such as, a halo group, for example, bromo, an alkyl- or arylsulphonyloxy group, such as, trifluoromethanesulphonyl, methanosulphonyl or p-toluenesulphonyl or a secondary acyclic or cyclic amino group, such as, dimethylamino or pyrrolidinyl), typically by treatment with a suitable nucleophilic reagent for example, in the case where X is azidomethyl, sodium azide in a polar solvent for example acetone at elevated temperature or wherein X is aminomethyl by treatment with ammonia under the same conditions.

Compounds of formula (II) wherein X is $C_{1-4}$ alkylthiomethyl, for example methylthiomethyl, may conveniently be prepared from a corresponding compound of formula (III) wherein $L^1$ is as hereinbefore defined for example, bromo, methanesulphonyl or pyrrolidinyl by treatment with sodium thiomethoxide. Similarly, compounds of formula (II) wherein X is $C_{1-4}$ alkoxymethyl, for example, methoxymethyl or ethoxymethyl may be prepared from compounds of formula (III) wherein $L^1$ is a suitable leaving group, for example, bromo, methanesulphonyl or pyrrolidinyl by treatment with methanol or ethanol respectively.

Compounds of formula (II) wherein X is a methyl group substituted by one or more halo atoms may conveniently be prepared by for example methods described by Matulic Adamic et al. 1988 J. Med.Chem. 31 1642-1647.

For example compounds of formula (II) wherein X is a halomethyl group for example fluoromethyl, may conveniently be prepared from compounds of formula (III) wherein $L^1$ is for example bromo or hydroxy typically in the case wherein $L^1$ is bromo by treatment with a fluorinating agent such as silver fluoride in a polar solvent, for example, acetonitrile at room temperature. In the case wherein $L^1$ is hydroxy by treatment with a fluorinating agent such as diethylaminosulphur trifluoride (DAST) in the presence of an inert solvent, for example dichloromethane (DCM) at reduced temperature.

Compounds of formula (III) wherein $L^1$ is hydroxy may conveniently be prepared from compounds of formula (III) wherein $L^1$ is halo typically in the presence of a base such as sodium hydrogen carbonate($NaHCO_3$) in an aqueous organic solvent for example aqueous tetrahydrofuran (THF) at room temperature.

Compounds of formula (II) wherein X is a dihalomethyl group, for example, difluoromethyl, may conveniently be prepared by fluorination of a compound of formula (II) wherein X is a formyl group by reaction with a fluorinating agent such as DAST in the presence of an inert solvent such as DCM at reduced temperature. This compound may itself be produced by oxidation of a compound of formula (III) wherein $L^1$ is hydroxy, typically by reaction with an oxidising agent for example tetrapropylammonium perruthenate (TPAP) with a co-oxidant such as 4-methylmorpholino-N-oxide in an inert solvent for example DCM at room temperature. Compounds of formula (III) wherein $L^1$ is hydroxy may be prepared as described above.

Compounds of formula (II) wherein X is a trihalomethyl group, for example, a trifluoromethyl group, may conveniently be prepared by treating a compound of formula (II) wherein X is a leaving group, such as, a halogen

atom, for example, iodine with the appropriate trihalomethyl precursor such as iodotrifluoromethane in the presence of a metal such as copper in a polar solvent such as hexamethylphosphoramide at elevated temperature.

Compounds of formula (II) wherein X is a halogen atom may be prepared by, for example, the method described by Robins et al 1982. Can. J. Chem 60, 554, that is by halogenating a compound of formula (II) wherein X is hydrogen, for example, by iodination using iodine monochloride, for example, in DCM, bromination using bromine, for example in glacial acetic acid and chlorination using a chlorine complex of iodobenzene, for example, in glacial acetic acid. Compounds of formula (II) wherein X is hydrogen may be prepared by blocking the 5'-position of a compound of formula (IV) wherein Y is hydrogen in conventional manner, for example, in the case of acyl blocking groups by treatment with an appropriate acyl halide, for example, chloride or an anhydride.

The compound of formula (IV) wherein Y is hydrogen may be prepared as described, for example, by Kowollick et al. J. Prackt. Chem. 1973 315(5) 895-900.

Compounds of formula (III) wherein OR is as hereinbefore defined and $L^1$ is a suitable leaving group other than hydroxy may be prepared by methods well known in the art, for example, in the case where $L^1$ is halogen by treatment of a compound of formula (II) wherein X is methyl with a suitable halogenating system, for example in the case where $L^1$ is to be bromo, N-bromosuccinimide in the presence of azobisisobutyronitrile (AIBN) and irradiation with u/v light.

Compounds of formula (III) where $L^1$ is an alkyl- or arylsulphonyloxy group may conveniently be prepared from the corresponding compound of formula (III) where $L^1$ is hydroxy by treatment with an appropriate sulphonyl halide, for example, in the case where $L^1$ is to be methanesulphonyl, methanesulphonyl chloride, typically under basic conditions. Compounds of formula (III) wherein $L^1$ is hydroxy may be prepared as described above or alternatively by the hydroxymethylation of compounds of formula (II) where X is hydrogen according to the method of Kahilainen et al, Acta Chemica Scandinavian B, 1985, 39 477.

Compounds of formula (III) wherein $L^1$ is a secondary amino group, may be prepared according to the method of Badman et al, J. Chem. Soc. Chem. Commun. 1987, 1732, from compounds of formula (II) wherein X is hydrogen and OR is hydroxy or a protected hydroxy group, by reaction with the appropriate amine, for example dimethylamine or pyrrolidine, in the presence of a formylating agent, for example, formaldehyde.

The leaving ability of the group $L^1$ in compounds of formula (III) wherein $L^1$ is a secondary amino group may be improved by quaternisation of the nitrogen atom of the amino group, for example, by reaction with an alkyl halide such as methyl iodide typically in an alcoholic solvent preferably methanol to produce the corresponding methiodide of the amine.

Compounds of formula (II) wherein X is hydrogen or methyl and OR is a protected hydroxy group may be prepared from the corresponding compounds of formula (II) wherein OR is hydroxy by treatment with an appropriate acyl halide, for example, the chloride, or anhydride to provide the corresponding ester or with an appropriate alkyl or silyl halide to give the corresponding ether or silylether.

The compound of formula (II) wherein X is hydrogen or methyl and OR is hydroxy may be prepared as described, for example, by Kowollick et al J. Prackt Chem. 1973 315 (5) 895.

The compound of formula (II) wherein X is nitro may conveniently be prepared by the method described by Huang and Torrence 1977 J. Org. Chem 42 (24) 3821, that is by treating a compound of formula (II) wherein X is hydrogen and R is a blocking group such as phosphate with a nitrating agent, for example, nitronium tetrafluoroborate in an inert solvent, for example, sulpholane at room temperature.

Compounds of formula (II) wherein X is hydrogen may conveniently be prepared by blocking the 5'-position of compounds of formula (IV) wherein Y is hydrogen in conventional manner, in the case of phosphate blocking groups by treatment, for example, with a phosphorylating agent such as phosphoryl chloride in a phosphate solvent such as triethyl phosphate at reduced temperature.

The corresponding compound of formula (IV) wherein Y is hydrogen may be prepared as described by Kowollick et at (1973).

Compounds of formula (II) wherein X is 2-haloalkynyl may conveniently be prepared by dehydrohalogenation of a compound of formula (II) wherein X is the corresponding symmetrical 1-2-dihalovinyl group typically by treatment with 1,8-diazabicyclo[5.4.0] undec-7-ene (DBU) in the presence of an inert solvent such as dichloromethane at room temperature. The 1-2-dihalovinyl precursor may conveniently be prepared by dihalogenation of a compound of formula (II) where X is the corresponding ethynyl group by reaction with a brominating agent for example tertiary butyl bromide in the presence of a polar aprotic solvent, for example, dimethylsulphoxide (DMSO) at room temperature.

The latter compound of formula (II) may be prepared by blocking the 5'-hydroxy group of the corresponding alcohol by treatment with an appropriate acyl halide, for example chloride or an anhydride in the presence of pyridine at room temperature. The said alcohol may be prepared, for example, as described in European Patent Application No. 88850370.3 or by analogy therewith.

Process B may be carried out, for example, by the method described by Lin et al. 1988 J.Med. Chem.31 336-340 i.e. by treating a compound of formula (IV) wherein Y is hydroxy with an alkylating agent such as alkyl halide, for example, methyl bromide in the presence of a base, such as sodium hydroxide in aqueous methanol at room temperature.

Compound of formula (IV) wherein Y is hydroxy may conveniently be prepared by hydroxylating a compound of formula (IV) wherein Y is halogen, for example, by treatment with triethylamine in aqueous methanol at room temperature. The latter compound of formula (IV) may be prepared by halogenating a compound of formula (IV) wherein Y is hydrogen, for example, using bromine in aqueous THF. Compounds of formula (IV) wherein Y is hydrogen may be prepared as above.

Process C may be carried out by treating a compound of formula (IV) wherein Y is a suitable leaving group, for example, chloromercuri with a thiolating reagent capable of replacing the leaving group with a $C_{1-4}$ alkylthio group, for example, in the case where Y is chloromercuri, $C_{1-4}$ dialkyldisulphide in the presence of a palladium catalyst such as Lithiumpalladium-tetrachloride ($Li_2PdCl_4$) in a polar solvent such as methanol.

The compound of formula (IV) where Y is a leaving group may be prepared from a compound of formula (IV) wherein Y is hydrogen, for example, in the case where Y is chloromercuri, by treatment with mercuric acetate in the presence of a chloride salt such as sodium chloride in a solvent, for example, water at elevated temperature.

The compound of formula (IV) wherein Y is hydrogen may be prepared as described above.

With regard to process (D), this may be effected for example by treatment of a compound of formula (V) in which Z represents a leaving group e.g. hydroxy or organosulphonyloxy such as methanesulphonyloxy or trifluoromethanesulphonyloxy with an appropriate fluorinating agent such as diethylaminosulphurtrifluoride, potassium fluoride, potassium hydrogen fluoride, or tetra-n-butylammonium fluoride.

Process (E) may be effected for example by treating the pyrimidine base of formula (VI) or a salt or protected derivative thereof, with 3'-deoxy-3'-fluorothymidine for example in the presence of an appropriate pentosyl transferring enzyme or an organic catalyst such as trimethylsilyltrifluoromethane sulphonate in a buffered aqueous solution.

Alternatively, compounds of formula (VI) wherein $R^1$ is as hereinbefore defined or a salt or protected derivative thereof may be chemically coupled with a suitably activated derivative of 2,3-dideoxy-3-fluoro ribose by methods well known in the art, for example, Nucleoside Analogues : Chemistry, Biology, Medical Application, Ed. R.T. Walker, E. de Clercq and F. Eckstein, NATO Advanced Study Instituted, Plenum press (1979). Suitably activated 2',3-dideoxy-3-fluoro ribose derivatives may be prepared according to the method of Asahi Glass as described in Japanese Patent Application No. JP0129390. Compounds of formula (VI) wherein $R^1$ is as hereinbefore defined or a salt or protected derivative thereof may be obtained commercially or prepared by methods analogous to those described in processes (A), (B) or (C) above for the preparation of compounds of formula (I) having corresponding $R^1$ substituents.

The compound of formula (I) may be converted into a pharmaceutically acceptable ester thereof by reaction with an appropriate esterifying agent, e.g. an acid halide or anhydride. The compound of formula (I) including esters thereof, may be converted into pharmaceutically acceptable salts thereof in conventional manner, e.g. by treatment with an appropriate base. An ester or salt of a compound of formula (I) may be converted into the parent compound, e.g. by hydrolysis.

The following Examples are intended for illustration only and are not intended to limit the scope of the invention in any way. The term 'active ingredient' as used in the Examples means a compound of formula (I) or a pharmaceutically acceptable derivative thereof.

According to a further aspect of the invention, there are also provided :

a) compounds of formula (IB) or pharmaceutically acceptable derivatives thereof for use in therapy ;

b) pharmaceutical formulations comprising a compound of formula (IA) or (IB) ; or a pharmaceutically acceptable derivative thereof, together with a pharmaceutically acceptable carrier therefor.

c) the use of a compound of formula (IA) or (IB) ; or a pharmaceutically acceptable derivative thereof in the manufacture of medicament for the treatment or prophylaxis of a retroviral infection, particularly a Human Immunodeficiency Virus or a hepatitis B viral infection.

d) a method for the treatment or prophylaxis of a viral infection, particularly retroviral or hepatitis B viral infections of a mammal including humans which comprises treating the mammal with a antivirally effective amount of a compound of formula (IA) or (IB) ; or a pharmaceutically acceptable derivative thereof.

The following Examples are provided by way of illustration of the present invention and should in no way be construed as limitations thereof.

Example 1

a) 2′,3′-Dideoxy-3′-fluoro-5-methyl-5′-0-(tert-butyldiphenylsilyl) uridine

To a stirred solution of 2′,3′-dideoxy-3′-fluoro-5-methyluridine (15g, 0.06mole) and tert-butylchlorodiphenylsilane (19.2ml) in dry DMF (210ml) under nitrogen, was added imidazole (8.4g, 0.12mole). Stirring was maintained at room temperature over night, the solvent was evaporated and the residue was partitioned between ethyl acetate/water (450ml each). The aqueous layer was further extracted with ethyl acetate (200ml), the combined organic fractions dried ($Na_2SO_4$) and evaporated to dryness. Trituration of the residue with ether, followed by storage at 0°C gave title compound as a white solid.

200MHz NMR δ($d_6$DMSO), 11.35(1H,bs,NH), 7.7-7.35(10H,m,aromatic Ms), 7.45(1H,s,H-6), 6.2(1H,dt,H-1′), 5.4(1H,dm,H-3′,$J_{3',F}$=54Hz), 4.25(1H,dt,H-4′,$J_{4',F}$=27.5), 3.87(2H,m,H-5′), 2.6-2.1(2H,m,H-2′), 1.5(3H,s,-$CH_3$), 1.05ppm(9H,s,tBu).

b) 2′,3′-Dideoxy-3′-fluoro-5-hydroxymethyl-5′-0-(tert-butyldiphenylsilyl) uridine

A solution of 2′,3′-dideoxy-3′-fluoro-5-methyl-5′-0-(tert-butyldiphenylsilyl) uridine (8g, 0.016mole) in dry $CCl_4$ (120mls) was added dropwise over a period of 20 mins. to a stirred refluxing suspension of N-bromosuccinimide (3.76g, 0.02mole) and AIBN (0.005g) in dry $CCl_4$ (50ml) that is being irradiated under nitrogen with a 100W tungsten bulb. When the addition was complete, the reaction mixture was stirred for a further 10 mins., cooled, filtered and the filtrate evaporated to dryness. The remaining residue was dissolved in THF (150ml) and $NaHCO_3$ (1.7g) in water (51ml) was added. The mixture was stirred at room temperature over night, concentrated and extracted with dichloromethane (3x30ml). Combined organic layers were dried ($Na_2SO_4$), evaporated to dryness and the residue was purified by column chromatography eluting with acetone/dichloromethane (3 :17) to give the title compound.

200MHz NMR δ($d_6$DMSO) 11.4(1H,bs,NH), 7.7-7.35(10H,m,aromatic H), 7.53(1H,s,H-6), 6.16(1H,dt,H-1′), 5.3(1H,dm,H-3′,$J_{3',F}$=54Hz), 4.83(1H,t,$CH_2\underline{OH}$), 4.2(1H,dt,H-4′,$J_{4',F}$=27Hz), 4.05(2H,d,$\underline{CH_2}OH$), 3.8(2H,m,H-5′), 2.65-2.1(2H,m,H-2′), 1.02ppm(9H,s,tBu).

c) 2′,3′-Dideoxy-3′-fluoro-5-fluoromethyl-5′-0-(tert-butyldiphenylsilyl)uridine

A solution of 2′,3′-dideoxy-3′-fluoro-5-hydroxymethyl-5′-0-(tert-butyldiphenylsilyl)uridine (0.8g, 1.6mmol) in dry $CH_2Cl_2$ (10ml) was added dropwise to a stirred solution of DAST (0.19ml, 1.6mmol) in dry $CH_2Cl_2$ (5ml) under $N_2$ at -15°C. The reaction mixture was stirred at -5°C for 30 mins. and room temperature for 1 hour. The mixture was poured onto ice/water (14ml), the organic layer separated, washed with water (10ml), dried ($Na_2SO_4$) and evaporated to dryness. The remaining residue was purified by column chromatography eluting with acetone/dichloromethane (3 :17 and 1 :16) to give the title compound (570mg, 71%).

200MHz NMR δ($d_6$DMSO) 11.6(1H,bs,NH), 7.9(1H,d,H-6), 7.7-7.35 (10H,m,aromatic H), 6.15(1H,dt,H-1′), 5.35(1H,dm,H-3′,$J_{3',F}$ =54Hz), 4.8(2H,d,$CH_2$F), 4.25(1H,dt,H-4′,$J_{4',F}$=27Hz), 3.88(2H,m, H-5′), 2.7-2.2(2H,m,H-2′), 1.03ppm(9H,s,tBu).

d) 2′,3′-Dideoxy-3′-fluoro-5-(fluoromethyl)uridine

A 1M solution of tetrabutylammonium fluoride in THF (1.6ml) was added dropwise to a stirred solution of 2′,3′-dideoxy-3′-fluoro-5-fluoromethyl-5′-0-(tert-butyldiphenylsilyl)uridine (0.33g,0.66 mmol) in THF (2.5ml) at 0°C and stirring was maintained for 40 mins. at 0°C and 10 mins. at room temperature. The reaction mixture was then applied directly onto a silica gel column packed in dichloromethane and eluted with tetrahydrofuran/dichloromethane (3 :7) to give the title compound.

200MHz NMR δ($d_6$DMSO) 11.58(1H,bs,NH), 8.15(1H,d,H-6), 6.18(1H,dt, H-1′), 5.32(1H,dd,H-3′), 5.15(1H,m,OH-5′), 5.06(2H,d,$\underline{CH_2}$F), 4.15(1H,m,H-4′), 3.61(2H,m,H-5′), 2.6-2.1ppm (2H,m,H-2′).
FAB Mass Spectrum : Observed $(M+Na)^+$285.

Example 2

2′,3′-Dideoxy-3′-fluoro-5-methoxymethyluridine

To a stirred solution of 2′,3′-dideoxy-3′-fluoro-5-fluoromethyl-5′-0-(tert-butyldiphenylsilyl) uridine (prepared as described in Example 1 (stage c) above) (0.55g, 1.1mmol) in dry THF (5ml) at 0°C was added a 1M solution

of tetrabutylammonium fluoride in THF (2.5ml). Stirring was maintained at 0°C for 45 mins, the mixture was evaporated to dryness and the residue purified by column chromatography eluting with methanol/dichloromethane (1 :19). The product fractions were concentrated and the solid recrystallised from methanol to give the title compound.

M.pt. 142-143°C

Microanalysis -

Calculated C,48.17 ; H,5.52 ; N,10.22%

Found C,48.13 ; H,5.4 ; N,9.82%

NMR δ($d_6$DMSO) 11.4(1H,bs,NH), 7.9(1H,s,H-6), 6.2(1H,dt,H-1'), 5.3(1H, dm,H-3',$J_{3',F}$=54Hz), 5.2(1H,m,OH-5'), 4.17(1H,dt,H-4',$J_{4',F}$=27Hz), 4.03(2H,s,$CH_2OCH_3$), 3.63(2H,m,H-5'), 3.23(3H,s,$CH_2O\underline{CH_3}$), 2.6-2.2ppm (2H,m,H-2').

Example 3

(a) 5'-0-Acetyl-2',3'-dideoxy-3'-fluoro-5-methyluridine

Acetic anhydride (6.2ml) was added dropwise to a stirred solution of 2',3'-dideoxy-3'-fluoro-5-methyluridine (11g, 45mmol) in dry pyridine (100ml) at 0°C. The mixture was then allowed to warm up to room temperature and stirring maintained for 48 hours. The reaction mixture was quenched by pouring over a mixture of ice/water and extracted with methylene chloride (3x80ml). The organic layers were combined, dried ($Na_2SO_4$) and evaporated to dryness to give a solid which was recrystallised from ethanol to give the title compound.

M.pt. 123°C.

200MHz NMR δ($d_6$DMSO) 11.35(1H,bs,NH), 7.45(1H,s,H-6), 6.2(1H,bt, H-1'), 5.3(1H,dd,H-3'), 4.3(1H,m,H-4'), 4.25(2H,m,H-5'), 2.58-2.3(2H,m,H-2'), 2.05(3H,s,OAc), 1.75ppm(3H,s,$CH_3$).

(b) 5'-0-Acetyl-5-bromomethyl-2',3'-dideoxy-3'fluorouridine

A solution of 5'-0-Acetyl-2',3'-dideoxy-3'-fluoro-5-methyluridine (0.286g, 1mmol), N-Bromosuccinimide (0.356g, 2mmol) and AIBN (0.005g) was stirred in dry dichloromethane (15ml) under relux for 4 hours under $N_2$. The cooled mixture was evaporated down to give the crude title compound which was used immediately without further purification.

(c) 2',3'-Dideoxy-5-ethoxymethyl-3'-fluorouridine

A solution of 5'-0-acetyl-5-bromomethyl-2',3'-dideoxy-3'-fluorouridine (0.36g, 1mmol) in dry ethanol (20ml) was stirred under reflux for 3 hours under $N_2$. After allowing to stand for 7 days, the solvent was evaporated to dryness and the residue purified by column chromatography eluting with ethyl acetate. The product fractions were concentrated to give the title compound.

M.pt. 162°C

200MHz NMR δ($d_6$DMSO) 11.45(1H,6s,NH), 7.9(1H,s,H-6), 6.25(1H,dt, H-1'), 5.35(1H,dd,H-3'), 5.15(1H,m,OH-5'), 4.19(1H,m,H-4'), 4.09(2H,s,$\underline{CH_2}CH_3$), 3.65(2H,m,H-5'), 3.45(2H,q,$O\underline{CH_2}CH_3$), 2.6-2.1-(2H,m,H-2'), 1.15(3H,t,$OCH_2CH_3$).

Mass Spectrum : Observed m/z 288.

Example 4

(a) 5-0-Acetyl-2',3'-dideoxy-3'-fluoro-5-methylthiomethyluridine

A mixture of 5'-0-acetyl-5-bromomethyl-2',3'-dideoxy-3'-fluorouridine (0.36g, 1mmol prepared according to Example 3b) and sodium thiomethoxide (84mg, 1.2mmol) was stirred in dry dioxan (25ml) under $N_2$ at ambient temperature for 1/2 hour. The reaction mixture was filtered and the filtrate evaporated to dryness. The remaining residue was purified by column chromatography eluting with ethyl acetate to give the title compound.

200MHz NMR δ($d_6$DMSO) 11.5(1H,6s,NH), 7.6(1H,s,H-6), 6.2(1H,dt, H-1'), 5.35(1H,dd,H-3'), 4.35(1H,m,H-4'), 4.2(2H,m,H-5'), 3.3(2H, m,$\underline{CH_2}CH_3$), 2.6-2.1(2H,m,H-2'), 2.08(3H,s,$O\underline{Ac}$), 2.00ppm(3H,s,$CH_2S$ $\underline{CH_3}$).

Mass Spectrum : Observed m/z 332

(b) 2',3'-Dideoxy-3'-fluoro-5-methylthiomethyluridine

A solution of sodium methoxide (0.1ml of 1M solution in methanol was added to a stirred solution of 5'-0-acetyl-2',3'-dideoxy 3'fluoro-5-methylthiomethyluridine (0.04g, 0.12mmol) in dry methanol (10ml) under $N_2$ at room temperature. After 45 minutes the reaction mixture was neutralised with DOWEX 50($H^+$) resin. The resin was filtered, washed with methanol and filtrate evaporated to dryness. The residue was purified by column chromatography eluting with ethyl acetate to give the title compound.

M.pt. 176-177°C

200MHz NMR δ($d_6$DMSO) 11.45(1H,bs,NH), 7.85(1H,s,H-6), 6.2(1H,dd, H-1'), 5.3(1H,dd,H-3'), 5.15(1H,m,OH-5'), 4.2(1H,m,H-4'), 3.6(2H, m,H-5'), 3.25(2H,s,$CH_2SCH_3$), 2.6-2.1(2H,m,H-2'), 2ppm(3H,s,$CH_2C$ $CH_3$).

Mass Spectrum : Observed m/z 290.

Pharmaceutical Formulations

In the following formulation Examples, the "Active Ingredient" may be any compound of formula (I) or a pharmaceutically acceptable derivative thereof, for example compounds of Examples 1 to 4.

Example 5 Tablet Formulations

The following formulations A, B and C are prepared by wet granulation of the ingredients with a solution of povidone, followed by addition of magnesium stearate and compression.

Formulation A

| | | mg/tablet | mg/tablet |
|---|---|---|---|
| (a) | Active ingredient | 250 | 250 |
| (b) | Lactose B.P. | 210 | 26 |
| (c) | Povidone B.P. | 15 | 9 |
| (d) | Sodium Starch Glycollate | 20 | 12 |
| (e) | Magnesium Stearate | 5 | 3 |
| | | 500 | 300 |

Formulation B

| | | mg/tablet | mg/tablet |
|---|---|---|---|
| (a) | Active ingredient | 250 | 250 |
| (b) | Lactose | 150 | - |
| (c) | Avicel PH 101 | 60 | 26 |
| (d) | Povidone B.P. | 15 | 9 |
| (e) | Sodium Starch Glycollate | 20 | 12 |
| (f) | Magnesium Stearate | 5 | 3 |
| | | 500 | 300 |

Formulation C

| | mg/tablet |
|---|---|
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients.

Formulation D

| | mg/capsule |
|---|---|
| Active Ingredient | 250 |
| Pregelatinised Starch NF15 | 150 |
| | 400 |

Formulation E

| | mg/capsule |
|---|---|
| Active Ingredient | 250 |
| Lactose | 150 |
| Avicel | 100 |
| | 500 |

Formulation F (Controlled Release Formulation)

The formulation is prepared by wet granulation of the following ingredients with a solution of povidone followed by the addition of magnesium stearate and compression.

| | | mg/tablet |
|---|---|---|
| (a) | Active Ingredient | 500 |
| (b) | Hydroxypropylmethylcellulose (Methocel K4M Premium) | 112 |
| (c) | Lactose B.P. | 53 |
| (d) | Povidone B.P.C. | 28 |
| (e) | Magnesium Stearate | 7 |
| | | 700 |

Drug release takes place over a period of about 6-8 hours and is complete after 12 hours.

Example 6 Capsule Formulations

Formulation A

A capsule formulation is prepared by admixing the ingredients of Formulation D in Example 3 above and filling into a two-part hard gelatin capsule.

Formulation B

| | | mg/capsule |
|---|---|---|
| (a) | Active ingredient | 250 |
| (b) | Lactose B.P. | 143 |
| (c) | Sodium Starch Glycollate | 25 |
| (d) | Magnesium Stearate | 2 |
| | | 420 |

Capsules are prepared by admixing the above ingredients and filling into a two-part hard gelatin capsule.

Formulation C

| | | mg/capsule |
|---|---|---|
| (a) | Active ingredient | 250 |
| (b) | Macrogol 4000 BP | 350 |
| | | 600 |

Capsules are prepared by melting the Macrogol 4000 BP, dispersing the active ingredient in the melt and filling the melt into a two-part hard gelatin capsule.

Formulation D

| | mg/capsule |
|---|---|
| Active ingredient | 250 |
| Lecithin | 100 |
| Arachis Oil | 100 |
| | 450 |

Capsules are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients (a), (b) and (c) using an extruder, followed by spheronisation of the extrudate and drying. The dried pellets are then coated with release-controlling membrane (d) and filled into a two-piece, hard gelatin capsule.

| | | mg/capsule |
|---|---|---|
| (a) | Active Ingredient | 250 |
| (b) | Microcrystalline Cellulose | 125 |
| (c) | Lactose BP | 125 |
| (d) | Ethyl Cellulose | 13 |
| | | 513 |

Example 7 Injectable Formulation

Formulation A

| | | |
|---|---|---|
| Active ingredient | 0.200g | |
| Hydrochloric acid solution, 0.1M | | q.s. to pH 4.0 to 7.0 |
| Sodium hydroxide solution, 0.1M | | q.s. to pH 4.0 to 7.0 |
| Sterile water | | q.s. to 10ml |

The active ingredient is dissolved in most of the water (35°-40°C) and the pH adjusted to between 4.0 and 7.0 with the hydrochloric acid or the sodium hydroxide as appropriate. The batch is then made up to volume with the water and filtered through a sterile micropore filter into a sterile 10ml amber glass vial (type 1) and sealed with sterile closures and overseals.

Formulation B

Active ingredient 0.125 g
Sterile, pyrogen-free, pH 7 phosphate buffer, q.s. to 25 ml

Example 8 Intramuscular injection

| | | |
|---|---|---|
| Active Ingredient | | 0.20 g |
| Benzyl Alcohol | | 0.10 g |
| Glycofurol 75 | | 1.45 g |
| Water for Injection | q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

Example 9 Syrup

| | | |
|---|---|---|
| Active ingredient | | 0.25 g |
| Sorbitol Solution | | 1.50 g |
| Glycerol | | 2.00 g |
| Sodium Benzoate | | 0.005 g |
| Flavour, Peach 17.42.3169 | | 0.0125 ml |
| Purified Water | q.s. to | 5.00 ml |

The active ingredient is dissolved in a mixture of the glycerol and most of the purified water. An aqueous solution of the sodium benzoate is then added to the solution, followed by addition of the sorbitol solution and finally the flavour. The volume is made up with purified water and mixed well.

Example 10 Suppository

| | mg/suppository |
|---|---|
| Active Ingredient | 250 |
| Hard Fat, BP (Witepsol H15 - Dynamit Nobel) | 1770 |
| | 2020 |

One-fifth of the Witepsol H15 is melted in a steam-jacketed pan at 45°C maximum. The active ingredient is sifted through a 200 μm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250 μm stainless steel screen and, with continuous stirring, is allowed to cool to 40°C. At a temperature of 38°C to 40°C, 2.02g of the mixture is filled into suitable, 2 ml plastic moulds. The suppositories are allowed to cool to room temperature.

Example 11 Pessaries

| | mg/pessary |
|---|---|
| Active ingredient | 250 |
| Anhydrate Dextrose | 380 |
| Potato Starch | 363 |
| Magnesium Stearate | 7 |
| | 1000 |

The above ingredients are mixed directly and pessaries prepared by direct compression of the resulting mixture.

Example 12 Antiviral and Toxicity Testing

(a) Antiviral MT4 Assay and Toxicity Testing

Antiviral activity against the Human Immunodeficiency Virus (HIV) was determined according to the method of Pauwels et al, J.Virol.Methods, 1988 20 309-321 by measuring the ability of the compound to reverse the cytopathic effect of HIV infection. This was determined by a quantitative assessment of cell growth monitored at the fifth day post infection by a tetrazolium dye (MTT) uptake test. Subconfluent (20-40,000 cells/well) human T lymphocyte cell line MT4 cells infected with HIV were grown in 96-well microtiter dishes and exposed to different dilutions of drug. After 5 days, the dye intake test was performed on drug treated cultures and on HIV infected and mock infected MT4 cells. Under the conditions of the test, HIV infection caused extensive cytopathic effect and prevented cell growth by >80%. The antiviral effect of a drug is reported as an IC-50, i.e. as the inhibitory concentration that would protect 50% of the cells from cell killing, measured as 50% of that cell growth determined for uninfected MT4 cell controls.

Cell toxicity was assessed in a cell growth inhibition assay on uninfected MT4 cells or on vero cells in a 96-well microtiter dish. Identical cell numbers of uninfected cells were exposed to different dilutions of drug and cell viability determined daily on replicate cultures using uptake of MTT. The concentration required for a 50% inhibition of cell viability at 5 days is termed CCID-50.

(b) HeLa-CD4$^{+}$ cell assay for evaluating susceptibility of HIV to antiviral compounds

Susceptibility of HIV to inhibitors was determined by infection of HT4-6C cell monolayers as described by Larder, B.A., Chesebro, B. & Richman, D.D. Antimicrob. Agents Chemother. 1990 34, 436-441. Briefly cells were seeded in 24-well multiwells at $5x10^4$ cells per well and incubated overnight at 37°C in growth medium (DMEM10). Monolayers were infected with 100-200pfu of cell-free virus in 0.2ml of DMEM containing 5% fetal bovine serum plus antibiotics (DMEM5) and incubated for 1 hour at 37°C to allow virus adsorption. Following this time, 0.8ml of DMEM5 (with or without inhibitor) was added to each well and cultures were incubated at 37°C for 2-3 days. Monolayers were fixed with 10% formaldehyde solution in PBS and stained with 0.25% crystal violet in order to visualize virus plaques. Individual foci of multinucleated gian cells (plaques) were apparent using this staining procedure. $ID_{50}$ values were derived from plots of percent plaque reduction versus inhibitor concentration.

(c) PBL Assay

HIV assays were carried out in human peripheral blood lymphocytes (PBL's) prepared by fractionation of fresh human plasma on Ficoll gradients as described by Hartmann H. et al. Aids Research and Human Retroviruses, 1988, 4 457-466. The isolated PBL's were stimulated with growth factors IL2 and PHA for 3 days before infection with HIV. Growth of virus was monitored, after 4 days incubation at 37°C, by quantitative ELISA assays of the capsid antigen, P24. A range of concentrations of compound of known molarity was incorporated into the medium. Yield of P24 at each concentration are expressed as a percentage of the control and a dose response curve is drawn. From this curve the 50% inhibitory concentration (IC50) is estimated.

| | IC$_{50}$ vs HIV-1 (μM) | | | TOX(μM) |
|---|---|---|---|---|
| Compound No. | MT-4 | PBL | HELA-CD4 | |
| 2',3'-dideoxy-3'-fluoro-5-methoxymethyluridine | 8.4,6.4 | 1.89 | 11.9 | >500 |
| 2',3'-dideoxy-3'-fluoro-5-methylthiomethyluridine | 6.4,0.25,1.3 | 0.16,0.94 | 4.2 | >200 |
| 2',3'-dideoxy-3'-fluoro-5-ethoxymethyluridine | 0.6,4.2 | 1.52,1.63 | 6.3 | >200 |

## Claims

1. Use of a compound of formula (I)

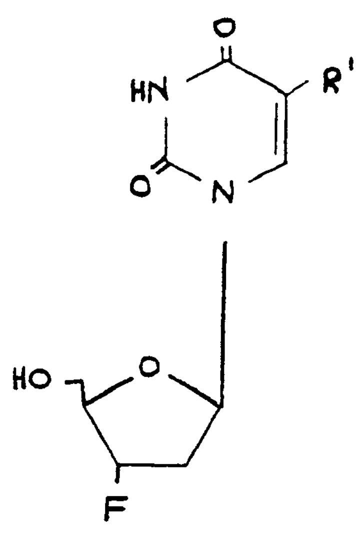

(I)

wherein $R^1$ represents a $C_{1-4}$alkylthiomethyl, $C_{1-4}$ alkoxymethyl, cyanomethyl, azidomethyl, mono-, di-, or tri-halomethyl, nitro, 2-haloalkynyl, aminomethyl, $C_{1-4}$ alkoxy or $C_{1-4}$ alkylthio group, or a pharmaceutically acceptable derivative thereof in the manufacture of a medicament for the treatment or prophylaxis of a retroviral infection.

2. Use of a compound of formula (I) as defined in claim 1 or a pharmaceutically acceptable derivative thereof, in the manufacture of a medicament for the treatment or prophylaxis of a human immunodeficiency virus infection.

3. Use of a compound of formula (I) as defined in claim 1 or a pharmaceutically acceptable derivative thereof, in the manufacture of medicament for the treatment or prophylaxis of a hepatitis B viral infection.

4. A compound of formula (IA)

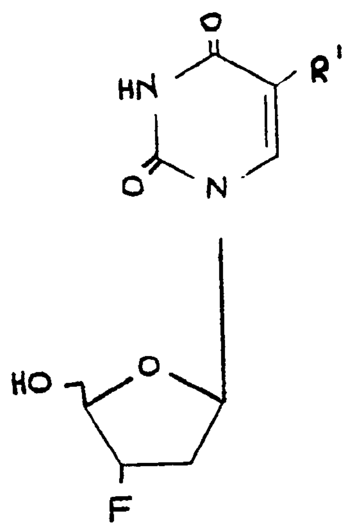

(IA)

wherein $R^1$ represents a $C_{1-4}$ alkylthiomethyl, $C_{1-4}$ alkoxymethyl, cyanomethyl, mono-, di- or tri-haloalkyl, nitro, 2-haloalkynyl, $C_{1-4}$ alkoxy or $C_{1-4}$ alkylthio group ; or a pharmaceutically acceptable derivative thereof for use in medical therapy.

5. A compound of formula (IB)

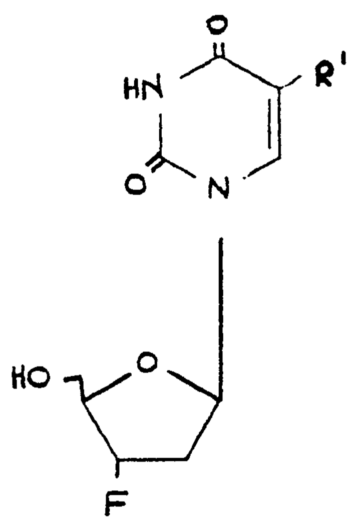

(IB)

wherein $R^1$ represents a $C_{1-4}$ alkylthiomethyl, $C_{1-4}$ alkoxymethyl, cyanomethyl, mono- di- or tri-halomethyl (other than bromomethyl), nitro, 2-haloalkynyl, $C_{1-4}$ alkoxy or $C_{1-4}$ alkylthio group ; or a pharmaceutical acceptable derivative thereof.

6. A compound of formula (IB) characterised in that $R^1$ represents a $C_{1-4}$ alkylthiomethyl or $C_{1-4}$ alkoxymethyl group ; or a pharmaceutically acceptable derivative thereof.

7. A compound of formula (IB) as defined in claim 5 or a pharmaceutically acceptable derivative thereof, which compound is selected from :-
2′,3′-Dideoxy-3′-fluoro-5-methoxymethyluridine ;
2′,3′-Dideoxy-3′-fluoro-5-ethoxymethyluridine ; or

2′,3′-Dideoxy-3′-fluoro-5-methylthiomethyluridine.

8. A compound as defined in any of claims 5 to 9, or a pharmaceutically acceptable derivative thereof for use in medical therapy.

9. Use of a compound as defined in any of claims 5 to 7, or a pharmaceutically acceptable derivative thereof in the manufacture of a medicament for the treatment or prophylaxis of a retroviral infection.

10. Use of a compound as defined in any of claims 5 to 7, or a pharmaceutically acceptable derivative thereof in the manufacture of a medicament for the treatment or prophylaxis of a human immunodeficiency virus infection.

11. Use of a compound as defined in any of claims 5 to 7, or a pharmaceutically acceptable derivative thereof in the manufacture of a medicament for the treatment or prophylaxis of a hepatitis B viral infection.

12. A pharmaceutical formulation comprising a compound as defined in any of claims 5 to 7, or a pharmaceutically acceptable derivative thereof, together with a pharmaceutically acceptable carrier therefor.

13. A process for the preparation of a compound of formula (IB) as claimed in claim 5, or a pharmaceutically acceptable derivative thereof which comprises :

(A) For the preparation of compounds of formula (IB) wherein $R^1$ represents a $C_{1-4}$alkylthiomethyl, $C_{1-4}$alkoxymethyl, cyanomethyl, mono-, di- or tri-halomethyl, nitro or 2-haloalkynyl group, removing a proteoting group from a compound of formula (II)

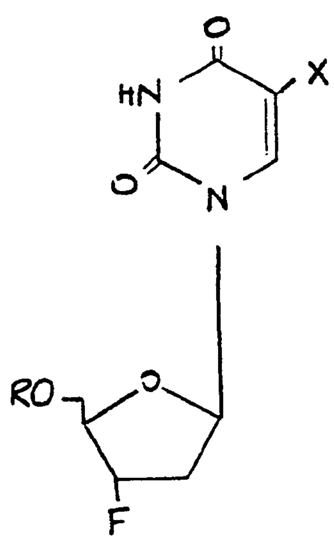

(II)

(wherein OR is a protected hydroxy group and X is the same as $R^1$ in the resulting compounds of formula (I) ;

(B) For the preparation of compounds of formula (IB) wherein $R^1$ represents a $C_{1-4}$ alkoxy group, reacting a compound of formula (IV)

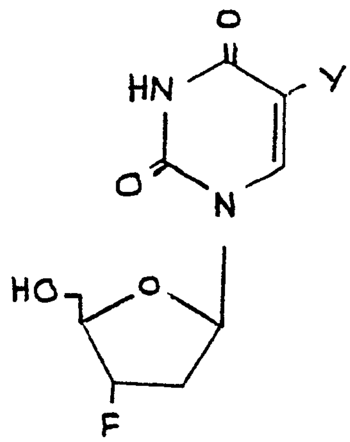

(IV)

(wherein Y is a hydroxy group) with an agent(s) and/or under such conditions as serve to alkylate the 5-hydroxy group on the uracil base.

(C) Process for the preparation of compounds of formula (IB) wherein $R^1$ represents a $C_{1-4}$ alkylthio group, reacting a compound of formula (IV) wherein Y represents a leaving group with an agent(s) and/or under such conditions serving to introduce a $C_{1-4}$ alkylthio group at the 5-position.

(D) reacting a compound of formula (V) :

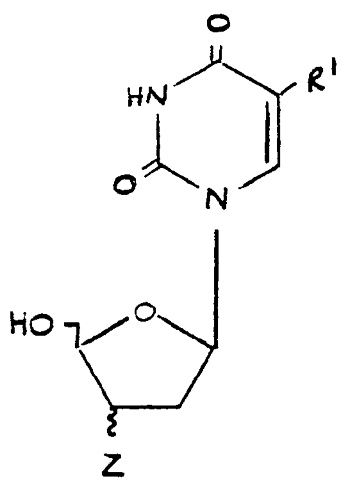

(V)

(wherein $R^1$ is as hereinbefore defined and Z represents a precursor group for the fluoro group) with an agent(s) and/or under such conditions serving to convert the said precursor group to a fluoro group; or

(E) reacting a pyrimidine base of formula (VI) :

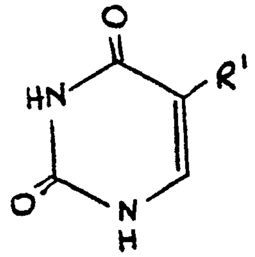

(VI)

(wherein $R^1$ is as hereinbefore defined) or a functional equivalent thereof, with a compound serving to introduce the desired ribofuranosyl ring at the 1-position of the pyrimidine base of formula (VI) ;

and thereafter, or simultaneously therewith, effecting one or more of the following optional conversions :-

(i) removing any remaining protecting groups ;

(ii) when a compound of formula (IB) is formed, converting it into a pharmaceutically acceptable derivative thereof ;
(iii) when a pharmaceutically acceptable derivative of a compound of formula (IB) is formed, converting the said derivative into a compound of formula (IB), or a different derivative thereof.

**Claims for the following Contracting States : ES - GR**

1. A process for the preparation of a compound of formula (IB)

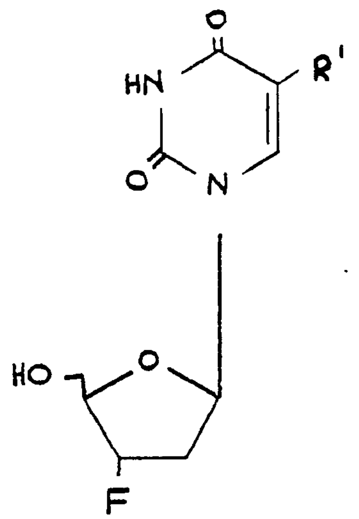

(IB)

wherein $R^1$ represents a $C_{1-4}$ alkylthiomethyl, $C_{1-4}$ alkoxymethyl, cyanomethyl, mono- di- or tri-halomethyl (other than bromomethyl), nitro, 2-haloalkynyl, $C_{1-4}$ alkoxy or $C_{1-4}$ alkylthio group ; or a pharmaceutical acceptable derivative thereof which comprises :

(A) For the preparation of compounds of formula (IB) wherein $R^1$ represents a $C_{1-4}$alkylthiomethyl, $C_{1-4}$alkoxymethyl, cyanomethyl, mono-, di- or tri-halomethyl (other than bromomethyl), nitro, 2-haloalkynyl group, removing a protecting group from a compound of formula (II)

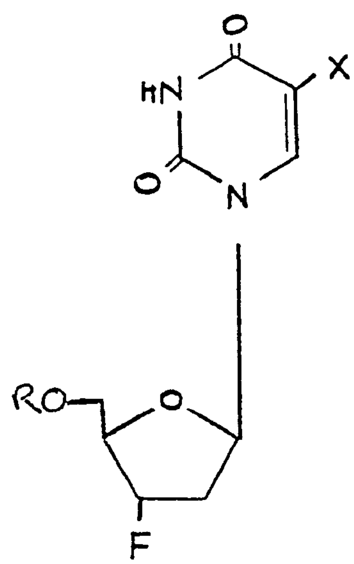

(II)

(wherein OR is a protected hydroxy group and X is the same as $R^1$ in the resulting compounds of formula (I) ;
(B) For the preparation of compounds of formula (IB) wherein $R^1$ represents a $C_{1-4}$ alkoxy group, reacting a compound of formula (IV)

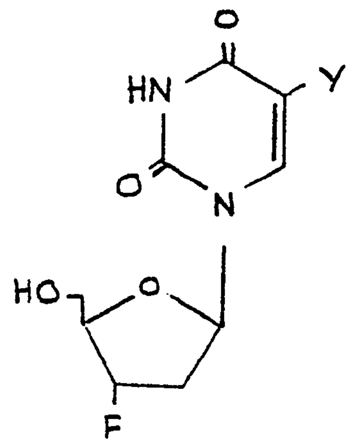

(IV)

(wherein Y is a hydroxy group) with an agent(s) and/or under such conditions as serve to alkylate the 5-hydroxy group on the uracil base.

(C) Process for the preparation of compounds of formula (IB) wherein $R^1$ represents a $C_{1-4}$ alkylthio group, reacting a compound of formula (IV) wherein Y represents a leaving group with an agent(s) and/or under such conditions serving to introduce a $C_{1-4}$ alkylthio group at the 5-position.

(D) reacting a compound of formula (V) :

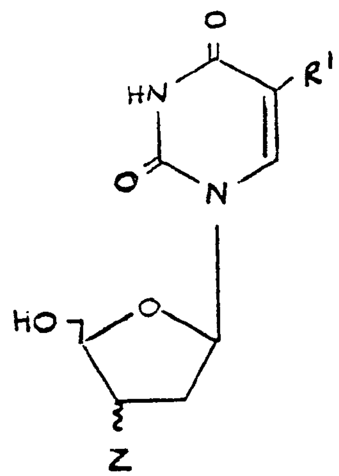

(V)

(wherein $R^1$ is as hereinbefore defined and Z represents a precursor group for the fluoro group) with an agent(s) and/or under such conditions serving to convert the said precursor group to a fluoro group; or

(E) reacting a pyrimidine base of formula (VI) :

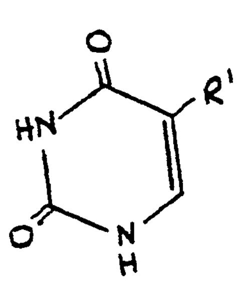

(VI)

(wherein $R^1$ is as hereinbefore defined) or a functional equivalent thereof, with a compound serving to introduce the desired ribofuranosyl ring at the 1-position of the pyrimidine base of formula (VI) ;

and thereafter, or simultaneously therewith, effecting one or more of the following optional conversions:-

(i) removing any remaining protecting groups ;

(ii) when a compound of formula (IB) is formed, converting it into a pharmaceutically acceptable derivative thereof ;
(iii) when a pharmaceutically acceptable derivative of a compound of formula (IB) is formed, converting the said derivative into a compound of formula (IB), or a different derivative thereof.

2. A process according to claim 1 for the preparation of a compound of formula (IB) characterised in that $R^1$ represents $C_{1-4}$ alkylthiomethyl or alkoxymethyl ; or a pharmaceutically acceptable derivative thereof.

3. A process for the preparation of a compound 2′,3′-dideoxy-3′-fluoro-5-methoxymethyluridine or a pharmaceutically acceptable derivative thereof.

4. A process for the preparation of a compound 2′,3′-dideoxy-3′-fluoro-5-ethoxymethyluridine or a pharmaceutically acceptable derivative thereof.

5. A process for the preparation of a compound 2′,3′-dideoxy-3′-fluoro-5-methylthiomethyluridine or a pharmaceutically acceptable derivative thereof.

6. A process for the preparation of a pharmaceutical formulation containing a compound of formula (IA)

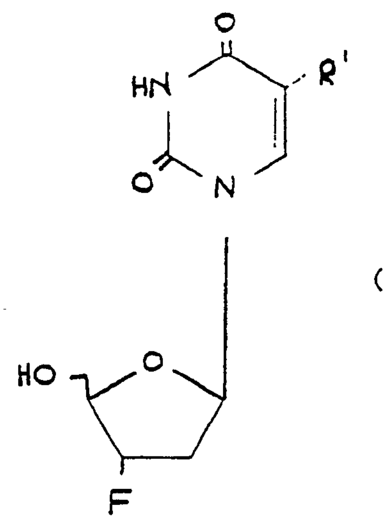

(IA)

wherein $R^1$ represents a $C_{1-4}$ alkylthiomethyl, $C_{1-4}$ alkoxymethyl, cyanomethyl, mono- di- or tri-halomethyl, nitro, 2-haloalkynyl, $C_{1-4}$ alkoxy or $C_{1-4}$ alkylthio group ; or a pharmaceutically acceptable derivative thereof which comprises preparing said compound by a process which comprises either :
(A) For the preparation of compounds of formula (IA wherein $R^1$ represents a $C_{1-4}$alkylthiomethyl, $C_{1-4}$alkoxymethyl, cyanomethyl, mono-, di- or tri-halomethyl, nitro, 2-haloalkynyl group, removing a protecting group from a compound of formula (II)

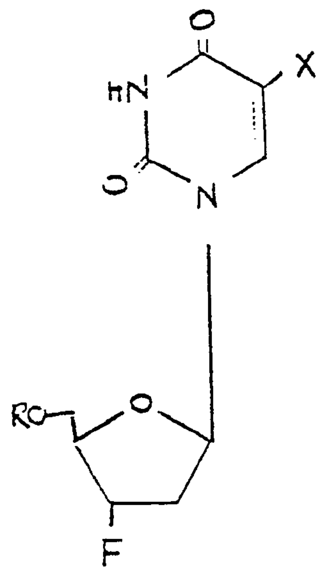

(II)

(wherein OR is a protected hydroxy group and X is the same as $R^1$ in the resulting compounds of formula (I) ;

(B) For the preparation of compounds of formula (IA) wherein $R^1$ represents a $C_{1-4}$ alkoxy group, reacting a compound of formula (IV)

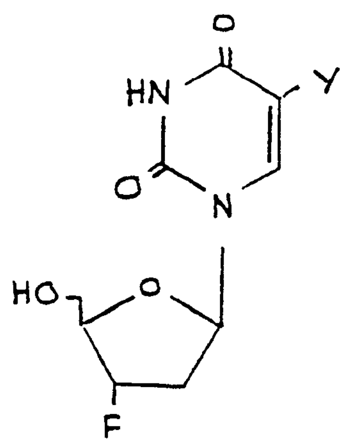

(IV)

(wherein Y is a hydroxy group) with an agent(s) and/or under such conditions as serve to alkylate the 5-hydroxy group on the uracil base.

(C) Process for the preparation of compounds of formula (IA) wherein $R^1$ represents a $C_{1-4}$ alkylthio group, reacting a compound of formula (IV) wherein Y represents a leaving group with an agent(s) and/or under such conditions serving to introduce a $C_{1-4}$ alkylthio group at the 5-position.

(D) reacting a compound of formula (V) :

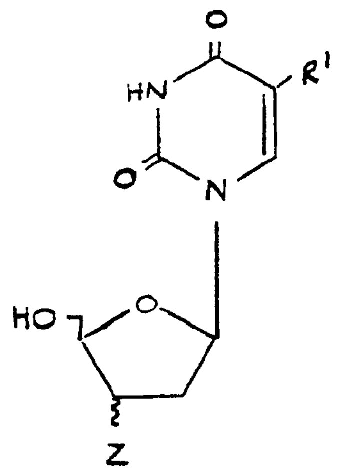

(V)

(wherein R¹ is as hereinbefore defined and Z represents a precursor group for the fluoro group) with an agent(s) and/or under such conditions serving to convert the said precursor group to a fluoro group; or

(E) reacting a pyrimidine base of formula (VI) :

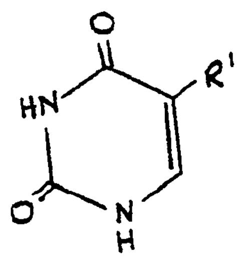

(VI)

(wherein R¹ is as hereinbefore defined) or a functional equivalent thereof, with a compound serving to introduce the desired ribofuranosyl ring at the 1-position of the pyrimidine base of formula (VI) ;

and thereafter, or simultaneously therewith, effecting one or more of the following optional conversions :-

(i) removing any remaining protecting groups ;

(ii) when a compound of formula (IB) is formed, converting it into a pharmaceutically acceptable derivative thereof ;

(iii) when a pharmaceutically acceptable derivative of a compound of formula (IB) is formed, converting the said derivative into a compound of formula (IB), or a different derivative thereof.

and bringing the resulting compound of formula (I) together with one or more pharmaceutically acceptable carriers to form the said pharmaceutical formlations.